# EUROPEAN PATENT APPLICATION

(11) **EP 4 101 467 A1**
(43) Date of publication of application: **14.12.2022**
(21) Application number: 21751414.0
(22) Date of filing: 03.02.2021
(51) Int. Cl.: A61K 45/00, A61P 1/18, A61P 41/00, A61P 43/00, A61K 9/06, A61K 9/10, A61K 9/107, A61K 47/10, A61K 47/34, A61K 47/36, A61K 47/38, A61K 47/42, A61K 31/17, A61K 31/365, A61K 31/536

(54) **AGENT FOR TREATING OR PREVENTING PANCREATIC FISTULA**

(30) Priority: 05.02.2020 JP 2020018309; 02.10.2020 JP 2020167881
(71) Applicant: NOF Corporation, Shibuya-ku Tokyo 150-6019 (JP)
(72) Inventor: UCHIDA, Yuichiro, Toyoake-shi, Aichi 470-1192 (JP); MASUI, Toshihiko, Kyoto-shi, Kyoto 606-8507 (JP); TABATA, Yasuhiko, Kyoto-shi, Kyoto 606-8507 (JP)
(74) Representative: Heubeck, Christian
(86) International application number: PCT/JP2021/003953
(87) International publication number: WO 2021/157619

(57) **Abstract**

The present invention provides a prophylactic or therapeutic agent for the leakage of pancreatic juice and/or pancreatic fistula after abdominal surgery, containing a lipase inhibitor as an active ingredient, and a method for preventing or treating leakage of pancreatic juice and/or pancreatic fistula after abdominal surgery, including administering an effective amount of a lipase inhibitor to a mammal in need of the administration thereof.

## Description

### [Technical Field]

The present invention relates to a prophylactic or therapeutic agent for the leakage of pancreatic juice or pancreatic fistula after abdominal surgery, and a method for preventing or treating the leakage of pancreatic juice or pancreatic fistula after abdominal surgery. The present invention is useful in the pharmaceutical field.

### [Background Art]

Pancreatic surgery is widely used clinically as the sole curative treatment for various pancreatic neoplastic lesions. After abdominal surgery, including pancreatic surgery, pancreatic juice may leak from the pancreas injured in relation to surgical operations. Pancreatic fistula is a serious complication after abdominal surgery which is caused by postoperatively-leaked pancreatic fluid which damages surrounding tissues, and is a major problem for the safe operation of pancreatic surgery and abdominal surgery dealing with the peripancreas.

Pancreatic fistula is classified into Biochemical leak, which has no clinically harmful effects, and Clinically relevant postoperative pancreatic fistula (CR-POPF), which is severe and requires various treatments (Non Patent Literature 1). However, it was not clear why postoperative leakage of pancreatic juice remains a biochemical leak in some patients, but it is aggravated into CR-POPF in some patients.

Therefore, a drug treatment specific for the leakage of pancreatic juice or pancreatic fistula after abdominal surgery has not been established yet, and establishment thereof has been awaited.

### [Citation List]

### [Non Patent Literature]

[NPL 1]
Surgery, 161(2017)584-591

### [Summary of Invention]

### [Technical Problem]

As described above, a prophylactic method and a drug treatment for the leakage of pancreatic juice and pancreatic fistula after abdominal surgery has not been established yet, and the development of a prophylactic or therapeutic agent exhibiting a high clinical effect is an urgent problem in the field of pharmaceutical technology.

### [Solution to Problem]

Under such circumstances, the present inventors have conducted intensive studies and found for the first time that a lipase inhibitor is useful for the prevention or treatment of the leakage of pancreatic juice and/or pancreatic fistula after abdominal surgery, and completed the present invention. Furthermore, they have also found a preparation containing a lipase inhibitor and suitable for administration to patients, which has solved the problem of water-insolubility of the lipase inhibitor.

Accordingly, the present invention provides the following.
[1] A prophylactic or therapeutic agent for the leakage of pancreatic juice and/or pancreatic fistula after abdominal surgery, comprising a lipase inhibitor as an active ingredient.
[2] The prophylactic or therapeutic agent of the above-mentioned [1] for the prophylaxis or treatment of pancreatic fistula after abdominal surgery.
[3] The prophylactic or therapeutic agent of the above-mentioned [2], wherein the pancreatic fistula is clinically relevant postoperative pancreatic fistula (CR-POPF).
[4] The prophylactic or therapeutic agent of any one of the above-mentioned [1] to [3], wherein the abdominal surgery is a pancreatic surgery.
[5] The prophylactic or therapeutic agent of any one of the above-mentioned [1] to [4], wherein the lipase inhibitor is one medicament or a combination of two or more medicaments, selected from orlistat, cetilistat, lalistat, and atglistatin.
[6] The prophylactic or therapeutic agent of the above-mentioned [5], wherein the lipase inhibitor is one medicament or a combination of two medicaments, selected from orlistat and cetilistat.
[7] The prophylactic or therapeutic agent of any one of the above-mentioned [1] to [6], wherein 0.01 to 20%(w/w) of the lipase inhibitor is contained.
[8] The prophylactic or therapeutic agent of any one of the above-mentioned [1] to [7], wherein the agent is for intraperitoneal administration (preferably, intraperitoneal local administration).
[9] The prophylactic or therapeutic agent of any one of the above-mentioned [1] to [8], wherein the agent is an emulsified preparation comprising a lipase inhibitor and a dispersing agent.
[10] The prophylactic or therapeutic agent of any one of the above-mentioned [1] to [8], wherein the agent is a hydrogel preparation comprising a complex of a lipase inhibitor and a hydrogel.
[11] The prophylactic or therapeutic agent of the above-mentioned [10], wherein the hydrogel is formed from any one of the following combinations of hydrogel-forming components:
   1) a combination of a hydrophilic biocompatible polymer and a biocompatible hydrophilic polymer crosslinking agent;
   2) a combination of a synthetic biocompatible polymer having a functional group (polymer A) and a synthetic biocompatible polymer having a functional group that reacts with polymer A under in vivo conditions (polymer B); or
   3) a combination of a low-molecular-weight compound having a functional group and a biocompatible hydrophilic polymer crosslinking agent.
[12] The prophylactic or therapeutic agent of the above-mentioned [11], wherein the synthetic biocompatible polymer comprises a polyalkylene oxide having a functional group.
[13] The prophylactic or therapeutic agent of the above-mentioned [11], wherein the hydrophilic biocompatible polymer is at least one selected from gelatin, collagen, fibrinogen, fibrin, cellulose, and chitosan, and derivatives thereof (more preferably, at least one selected from crosslinked gelatin, fibrinogen, and fibrin, and derivatives thereof, particularly preferably, at least one selected from fibrinogen and fibrin, and derivatives thereof).
[14] The prophylactic or therapeutic agent of any one of the above-mentioned [10] to [13], wherein 0.01 to 10%(w/w) of the lipase inhibitor is contained.
[15] The prophylactic or therapeutic agent of the above-mentioned [14], wherein 0.01 to 5% (lipase inhibitor weight/hydrogel-forming component weight) of the lipase inhibitor is contained.
[16] The prophylactic or therapeutic agent of any one of the above-mentioned [10] to [15], further comprising a dispersing agent.
[17] A method for preventing or treating leakage of pancreatic juice and/or pancreatic fistula after abdominal surgery, comprising administering an effective amount of a lipase inhibitor to a mammal in need of the administration thereof.
[18] The method of the above-mentioned [17], which is a method for preventing or treating pancreatic fistula after abdominal surgery.
[19] The method of the above-mentioned [18], wherein the pancreatic fistula is clinically relevant postoperative pancreatic fistula (CR-POPF).
[20] The method of any one of the above-mentioned [17] to [19], wherein the abdominal surgery is a pancreatic surgery.
[21] The method of any one of the above-mentioned [17] to [20], wherein the lipase inhibitor is one medicament or a combination of two or more medicaments, selected from orlistat, cetilistat, lalistat, and atglistatin.
[22] The method of the above-mentioned [21], wherein the lipase inhibitor is one medicament or a combination of two medicaments, selected from orlistat and cetilistat.
[23] The method of any one of the above-mentioned [17] to [22], wherein the lipase inhibitor is administered in the form of a pharmaceutical composition comprising 0.01 to 20%(w/w) of the lipase inhibitor.
[24] The method of any one of the above-mentioned [17] to [23], wherein the lipase inhibitor is intraperitoneally administered (preferably, intraperitoneally and locally administered).
[25] The method of any one of the above-mentioned [17] to [24], wherein the lipase inhibitor is administered in the form of an emulsified preparation comprising a lipase inhibitor and a dispersing agent.
[26] The method of any one of the above-mentioned [17] to [24], wherein the lipase inhibitor is administered in the form of a hydrogel preparation comprising a complex of a lipase inhibitor and a hydrogel.
[27] The method of the above-mentioned [26], wherein the hydrogel is formed from any one of the following combinations of hydrogel-forming components:
   1) a combination of a hydrophilic biocompatible polymer and a biocompatible hydrophilic polymer crosslinking agent;
   2) a combination of a synthetic biocompatible polymer having a functional group (polymer A) and a synthetic biocompatible polymer having a functional group that reacts with polymer A under in vivo conditions (polymer B); or
   3) a combination of a low-molecular-weight compound having a functional group and a biocompatible hydrophilic polymer crosslinking agent.
[28] The method of the above-mentioned [27], wherein the synthetic biocompatible polymer comprises a polyalkylene oxide having a functional group.
[29] The method of the above-mentioned [27], wherein the hydrophilic biocompatible polymer is at least one selected from gelatin, collagen, fibrinogen, fibrin, cellulose, and chitosan, and derivatives thereof (more preferably, at least one selected from crosslinked gelatin, fibrinogen, and fibrin, and derivatives thereof, particularly preferably, at least one selected from fibrinogen and fibrin, and derivatives thereof).
[30] The method of any one of the above-mentioned [26] to [29], wherein the hydrogel preparation comprises 0.01 to 10%(w/w) of the lipase inhibitor.
[31] The method of the above-mentioned [30], wherein the hydrogel preparation comprises 0.01 to 5% (lipase inhibitor weight/hydrogel-forming component weight) of the lipase inhibitor.
[32] The method of any one of the above-mentioned [26] to [31], wherein the hydrogel preparation further comprises a dispersing agent.
[33] A hydrogel preparation comprising a complex of a lipase inhibitor and a hydrogel.
[34] The hydrogel preparation of the above-mentioned [33], wherein the hydrogel is formed from any one of the following combinations of hydrogel-forming components:
   1) a combination of a hydrophilic biocompatible polymer and a biocompatible hydrophilic polymer crosslinking agent;
   2) a combination of a synthetic biocompatible polymer having a functional group (polymer A) and a synthetic biocompatible polymer having a functional group that reacts with polymer A under in vivo conditions (polymer B); or
   3) a combination of a low-molecular-weight compound having a functional group and a biocompatible hydrophilic polymer crosslinking agent.
[35] The hydrogel preparation of the above-mentioned [34], wherein the synthetic biocompatible polymer comprises a polyalkylene oxide having a functional group.
[36] The hydrogel preparation of the above-mentioned [34], wherein the hydrophilic biocompatible polymer is at least one selected from gelatin, collagen, fibrinogen, fibrin, cellulose, and chitosan, and derivatives thereof (more preferably, at least one selected from crosslinked gelatin, fibrinogen, and fibrin, and derivatives thereof, particularly preferably, at least one selected from fibrinogen and fibrin, and derivatives thereof).
[37] The hydrogel preparation of any one of the above-mentioned [33] to [36], wherein 0.01 to 10%(w/w) of the lipase inhibitor is contained.
[38] The hydrogel preparation of the above-mentioned [37], wherein 0.01 to 5% (lipase inhibitor weight/hydrogel-forming component weight) of the lipase inhibitor is contained.
[39] The hydrogel preparation of any one of the above-mentioned [33] to [38], further comprising a dispersing agent.

### [Advantageous Effects of Invention]

According to the present invention, a medicament that can be used effectively and safely for the prophylaxis or treatment of leakage of pancreatic juice or pancreatic fistula after abdominal surgery; and a method for preventing or treating the leakage of pancreatic juice or pancreatic fistula after abdominal surgery that uses the medicament; can be provided.

### [Brief Description of Drawings]

[Fig. 1]

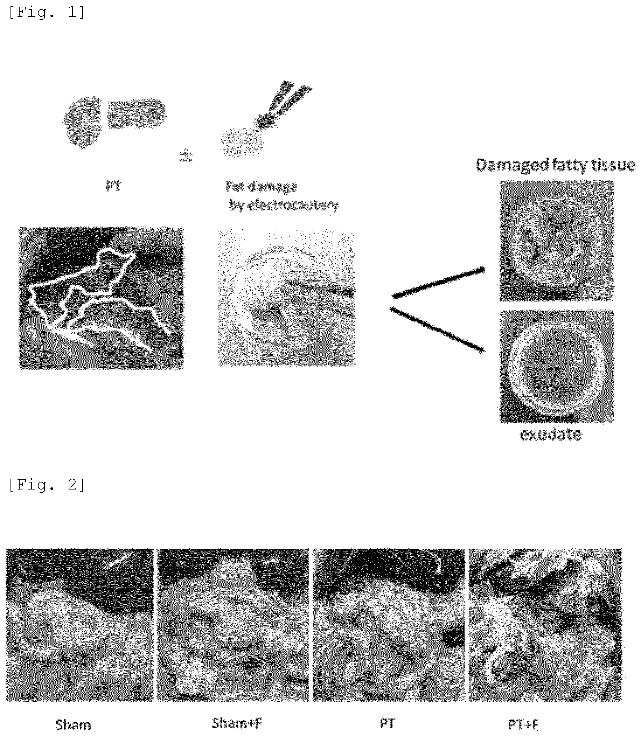

   Fig. 1 shows schematic views of the preparation of a pancreatic fistula model mouse (PT) and a fat damage treatment (+F) .
[Fig. 2]
   Fig. 2 shows intraperitoneal findings (24 hr after treatment) of each group studied in Example 1(1).
[Fig. 3]

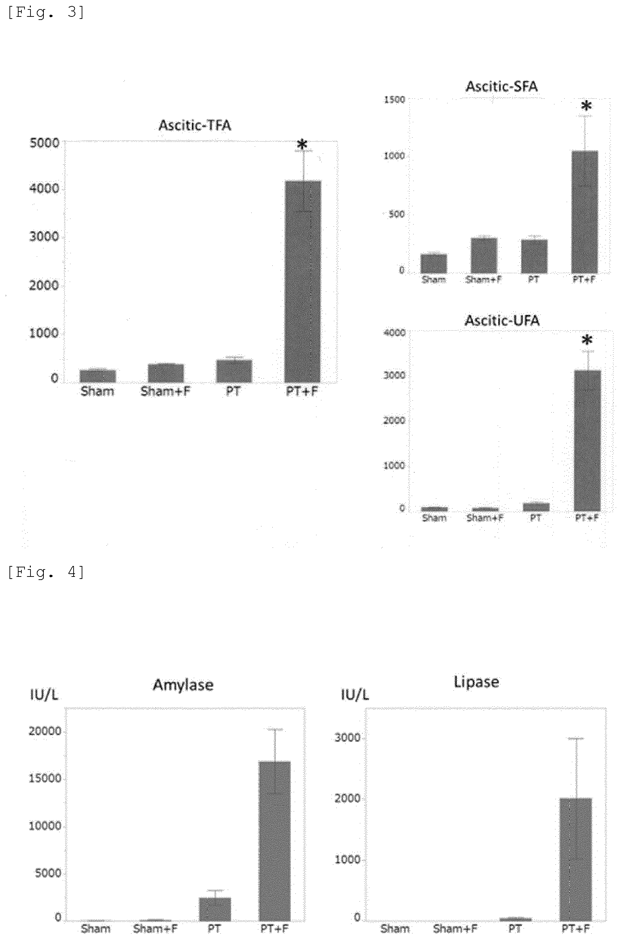

   Fig. 3 shows ascitic free fatty acid concentration of each group studied in Example 1(2). The significant test was performed with the Sham group by using the Tukey test. In the Figure, TFA means total free fatty acid, SFA means saturated free fatty acid, and UFA means unsaturated free fatty acid. Specific values of the TFA concentration (µM) were the following. Sham group: 263, Sham+F group: 390, PT group: 528, PT+F group: 4145.
[Fig. 4]
   Fig. 4 shows ascitic pancreatic enzyme activity (IU/L) of each group studied in Example 1(3). Specific activity of each enzyme was as follows. Amylase: Sham group: 34, Sham+F group: 104, PT group: 2468, PT+F group: 16868*) PT+F group showed a significantly higher enzyme activity than PT group (p=0.009; Mann Whitney U test). Lipase: Sham group: 0, Sham+F group: 2, PT group: 46, PT+F group: 2017*) PT+F group showed a significantly higher enzyme activity than PT group (p=0.009; Mann; Whitney U test).
[Fig. 5]

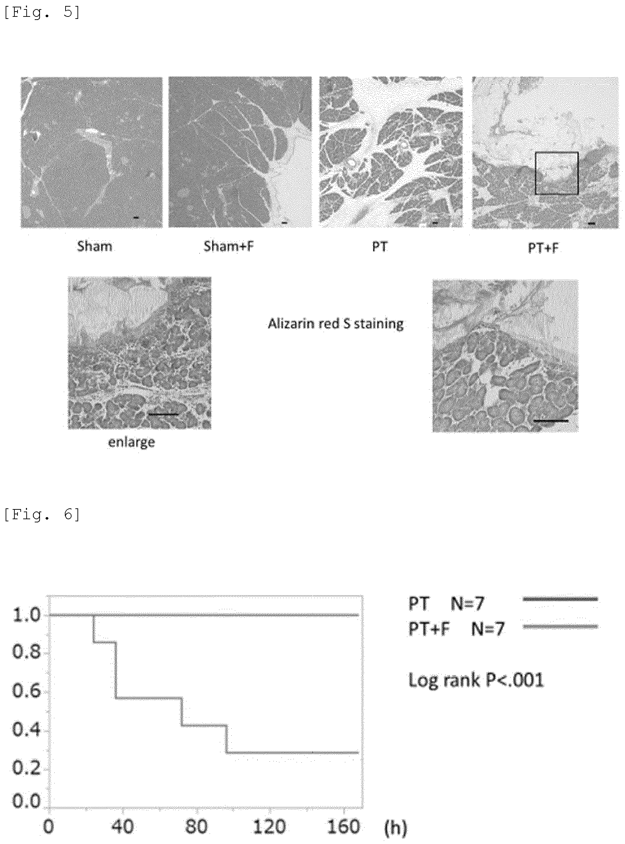

   Fig. 5 shows histological findings of each group studied in Example 1(4). Sham+F group did not show clear changes in the pancreas compared to the Sham group. In the PT group, mild edema of stroma and cell infiltration and the like were observed, and mild inflammatory changes were confirmed. In the PT+F group, in addition to those changes, deposition of glass-like substance was observed on the surface of the pancreas. The same part was stained orange by alizarin staining, indicating that it was deposition of fatty acid calcium. A high degree of inflammatory cell infiltration was observed around the glass-like deposits and an image of acinar cell damage was observed.
[Fig. 6]
   Fig. 6 shows the survival rates of the PT group and PT+F group studied in Example 1(5).
[Fig. 7]

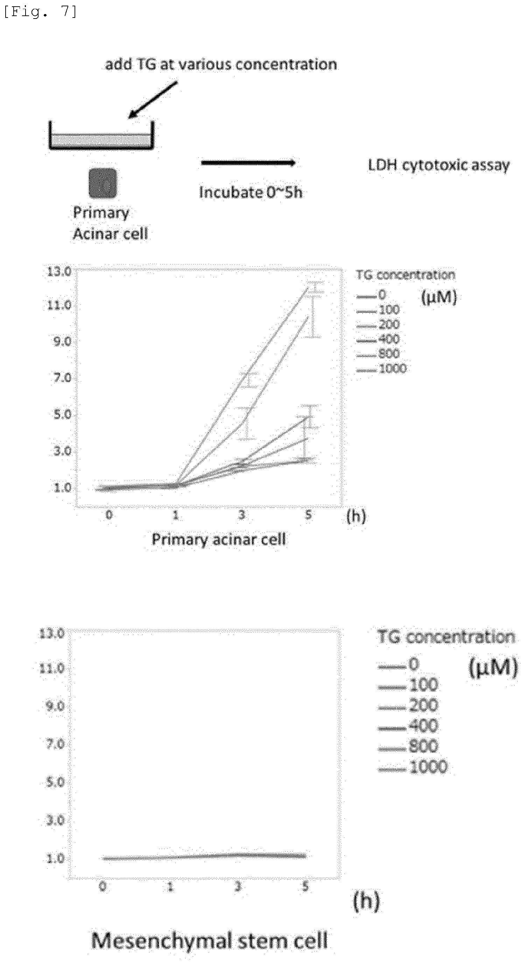

   Fig. 7 shows the effect of neutral fats on rat pancreatic acinar cells studied in Example 2(1).
[Fig. 8]

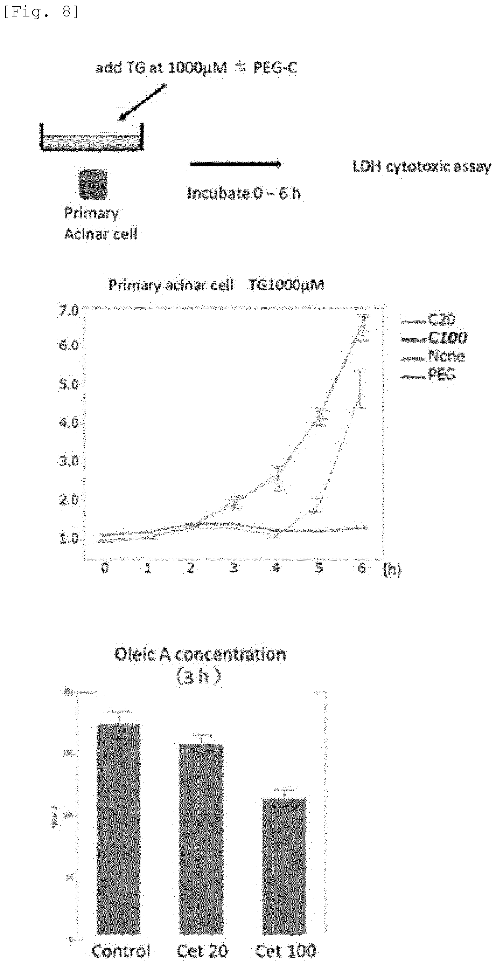

   Fig. 8 shows the pancreatic acinar cell-protecting effect of PEG-C studied in Example 2(2). In the Figure, C20 means a PEG-C 20 µM administration group, C100 means a PEG-C 100 µM administration group, None means a PEG-C non-administration group, and PEG means a PEG aqueous solution (amount equal to PEG-C 100 µM) administration group.
[Fig. 9]

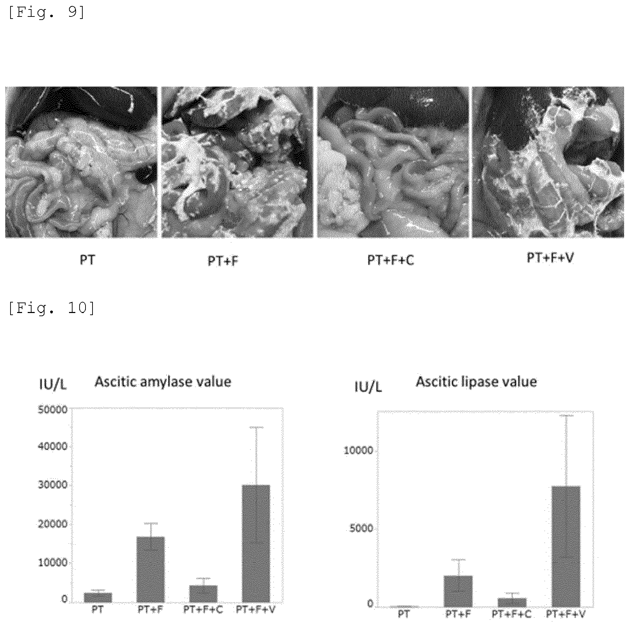

   Fig. 9 shows intraperitoneal findings (24 hr after treatment) of each group studied in Example 3(1).
[Fig. 10]
   Fig. 10 shows ascitic pancreatic enzyme activity (IU/L) of each group studied in Example 3(2). Specific activity of each enzyme was as follows. Amylase: PT group: 2468, PT+F group: 16868, PT+F+C group: 4374, PT+F+V group: 30120*) PT+F group showed a significantly higher enzyme activity than PT+F+C group (p=0.009; Mann Whitney U test). Lipase: PT group: 46, PT+F group: 2017; PT+F+C group: 573, PT+F+V group: 7725*) PT+F group showed a significantly higher enzyme activity than PT+F+C group (p=0.079; Mann; Whitney U test).
[Fig. 11]

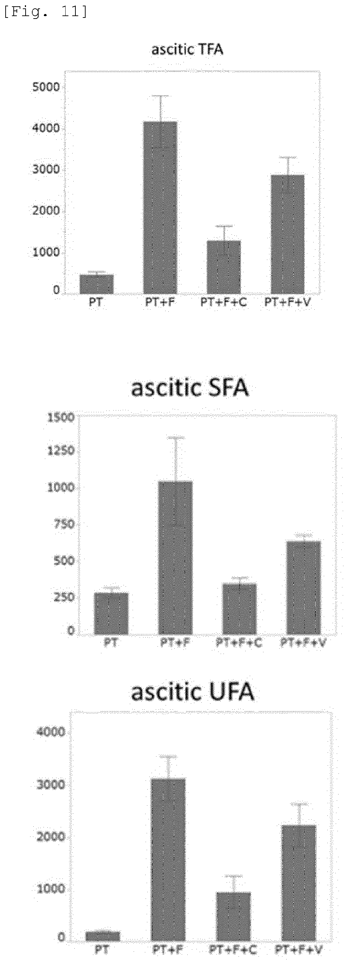

   Fig. 11 shows ascitic free fatty acid concentration (µM) of each group studied in Example 3(3). In the Figure, TFA means total free fatty acid, SFA means saturated free fatty acid, and UFA means unsaturated free fatty acid. Specific values of the TFA concentration (µM) were the following. PT group: 471, PT+F group: 4172, PT+F+C group: 1294, PT+F+V group: 2877*) PT+F group showed a significantly higher enzyme activity than PT+F+C group (p=0.016; Mann Whitney U test).
[Fig. 12]

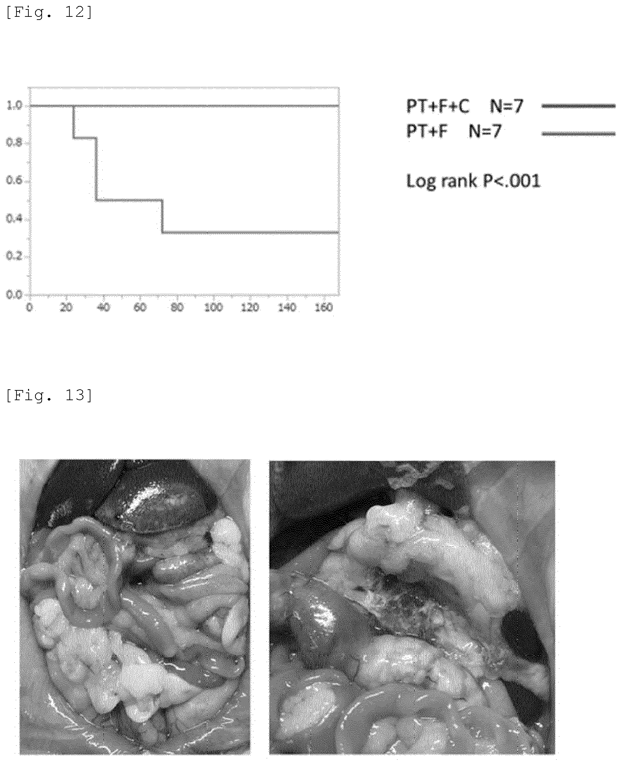

   Fig. 12 shows the survival rate of each group studied in Example 3(4).
[Fig. 13]
   Fig. 13 shows histological findings studied in Example 5(1) (PEG gel-C administration group). Attachment of a small amount of saponified substance is observed around the pancreas (around the hydrogel), but suppression of saponification is observed as a whole. Saponification is clearly suppressed as compared with the non-treatment group (PT+F group).
[Fig. 14]

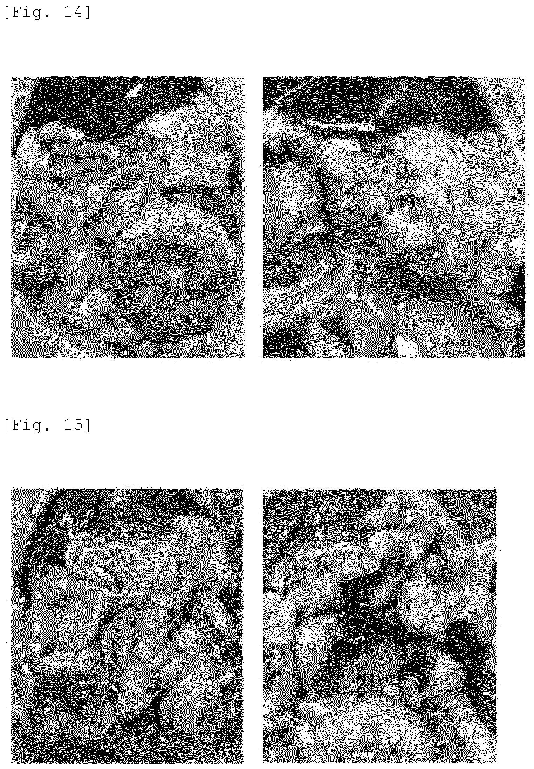

   Fig. 14 shows histological findings studied in Example 5(1) (PEG gel-C administration group). Attachment of a small amount of saponified substance is observed around the pancreas (around the hydrogel), but suppression of saponification is observed as a whole. Saponification is clearly suppressed as compared with the non-treatment group (PT+F group).
[Fig. 15]
   Fig. 15 shows histological findings studied in Example 5(1) (PEG gel administration group). A wide range of saponification and hemolysis findings were observed. Aggravation was seen.
[Fig. 16]

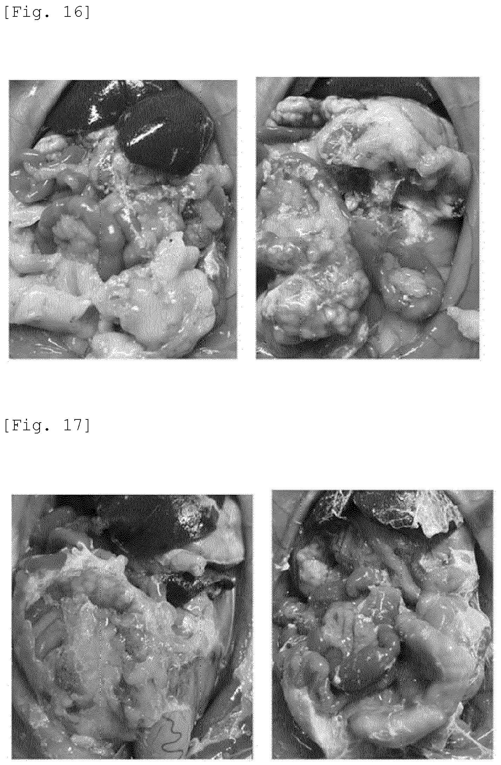

   Fig. 16 shows histological findings studied in Example 5(1) (PEG gel administration group). Saponification was confirmed to be prominent.
[Fig. 17]
   Fig. 17 shows the examination results of the histological findings in Example 5(1) (PEG gel-C administration group/PEG gel administration group) and the histological findings in the non-treatment group (PT+F group) as a comparison control. A wide range of saponification is observed in the abdominal cavity.
[Fig. 18]

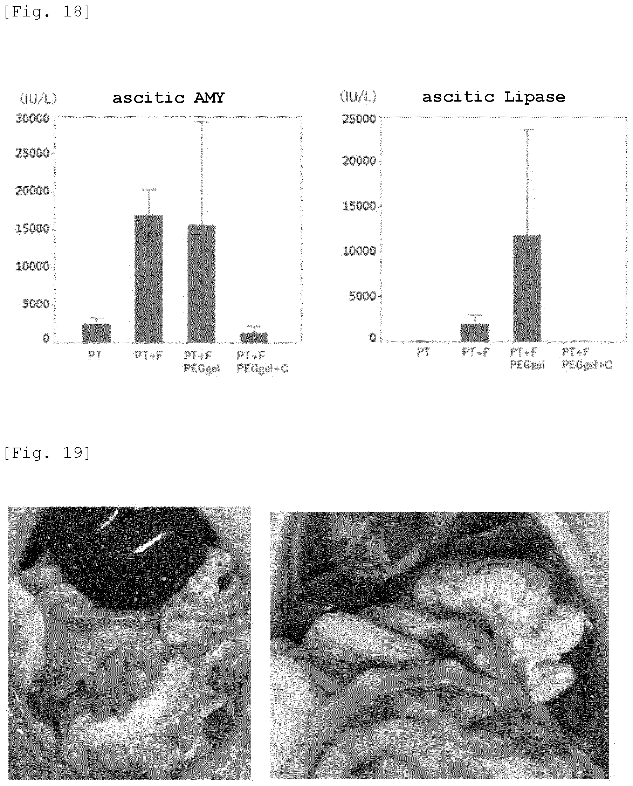

   Fig. 18 shows the examination results of the ascitic pancreatic enzyme activity (IU/L) in Example 5(2). In the Figure, ascitic AMY shows the enzyme activity of amylase, and ascitic Lipase shows the enzyme activity of lipase. Amylase: PT+F group: 16868, PEG gel-C group: 1278 (p=0.025; Mann-Whitney U test). Lipase: PT+F group: 2017, PEG gel-C group: 52 (p=0.025; Mann-Whitney U test).
[Fig. 19]

   Fig. 19 shows histological findings studied in Example 7(1) (PEG gel-O administration group). In the PEG gel-O group, a clear reduction (suppression) was confirmed in both the inflammatory findings and saponification in the entire abdominal cavity as compared with the non-treatment group (see Fig. 17).
[Fig. 20]

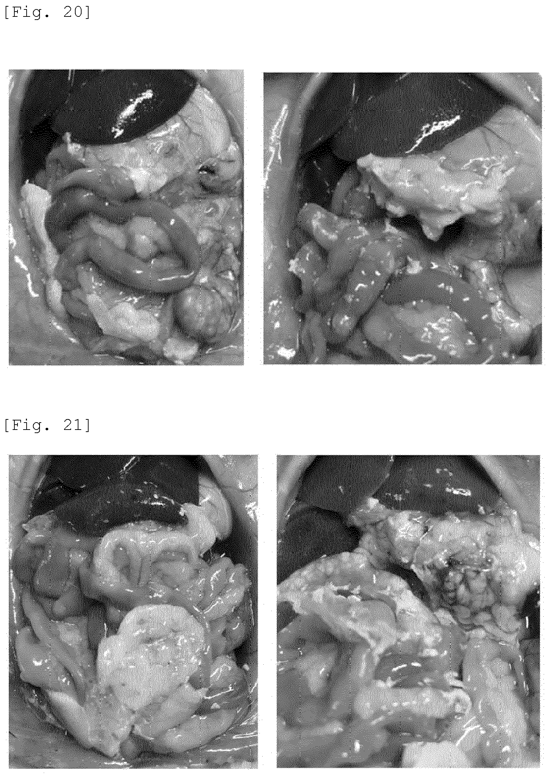

   Fig. 20 shows histological findings studied in Example 9(1) (bolheal-C administration group). In the bolheal-C group, a clear reduction (suppression) was confirmed in both the inflammatory findings and saponification in the entire abdominal cavity as compared with the non-treatment group (see Fig. 17).
[Fig. 21]
   Fig. 21 shows histological findings studied in Comparative Example 2(1) (bolheal alone administration group). In the bolheal alone administration group, the inflammatory findings and saponification in the entire abdominal cavity which are similar to those in the non-treatment group (see Fig. 17) were confirmed.

### [Description of Embodiments]

The present invention relates to a prophylactic or therapeutic agent for the leakage of pancreatic juice and/or pancreatic fistula after abdominal surgery, comprising a lipase inhibitor as an active ingredient; a method for preventing or treating leakage of pancreatic juice and/or pancreatic fistula after abdominal surgery, comprising administering an effective amount of a lipase inhibitor to a mammal in need of the administration thereof; a lipase inhibitor for use in the prophylaxis or treatment of leakage of pancreatic juice and/or pancreatic fistula after abdominal surgery; use of a lipase inhibitor for the production of a prophylactic or therapeutic drug for leakage of pancreatic juice and/or pancreatic fistula after abdominal surgery; and a pharmaceutical composition containing a lipase inhibitor useful for the practice of the present invention.

While the present invention mentioned above is described in detail in the following, unless otherwise specified, all technical terms and scientific terms used in the present specification have the same meanings as those generally understood by those skilled in the art to which the present invention belongs.

### (lipase inhibitor)

In the present specification, the "lipase inhibitor" is a general term for compounds that have an action of inhibiting the decomposition of neutral fats into free fatty acids by lipolytic enzymes (lipases) contained in pancreatic juice, and many "lipase inhibitors" are already known in this field of the art. The term "lipase inhibitor" has been recognized and used by those skilled in the art as a comprehensive term for this group of compounds. Preferred examples include Orlistat, Cetilistat, Lalistat, Atglistatin, and the like, and orlistat and cetilistat are more preferred.

For example, orlistat can be produced by the method described in JP-A-60-13777. Those of ordinary skill in the art can also obtain cetilistat, lalistat, and atglistatin by appropriately synthesizing them using a method known in this field of the art.

As demonstrated in the Example described later in which orlistat and cetilistat were used as representative "lipase inhibitors", the "lipase inhibitor" exerts a therapeutic effect on pancreatic fistula by suppressing the decomposition of neutral fats into free fatty acids in the abdominal cavity by lipase (lipolysis enzyme) leaked from the pancreas, based on the function of the lipase inhibitor to inhibit the decomposition of neutral fats into free fatty acids.

In practicing the present invention, the lipase inhibitor can be used in either a free form or in the form of a pharmaceutically acceptable salt thereof. Those of ordinary skill in the art can appropriately select from the both forms and practice the present invention, based on the characteristics of the individual lipase inhibitors to be used.

Examples of the pharmaceutically acceptable salt include a salt with an acid such as a salt with an inorganic acid (e.g., hydrochloride, hydrobromic acid salt, sulfuric acid salt, phosphate, or the like), a salt with an organic acid (e.g., acetate, fumarate, oxalate, citrate, methanesulfonic acid salt, benzenesulfonic acid salt, tosylic acid salt, maleate, or the like); and a salt with a base such as an alkali metal salt (e.g., sodium salt, potassium salt, or the like), an alkaline earth metal salt (e.g., calcium salt, or the like); a salt with an amino acid such as glycine salt, lysine salt, arginine salt, ornithine salt, glutamate, aspartate; and the like.

### (target disease)

In the present specification, the "abdominal surgery" in the "leakage of pancreatic juice or pancreatic fistula after abdominal surgery" includes surgery on the pancreas, stomach, duodenum, spleen, kidney, adrenal gland, large intestine, and the like. In particular, leakage of pancreatic juice and/or pancreatic fistula after pancreatic surgery or after gastric surgery (particularly after pancreatic surgery) is the application target in the present invention.

In the present specification, the "leakage of pancreatic juice" refers to an intraperitoneal leakage of pancreatic juice from the pancreas that occurs after abdominal surgery.

In the present specification, the "pancreatic fistula" refers to a complication caused by leaked pancreatic juice damaging the surrounding tissues, and includes those that do not have a clinically harmful effect (Biochemical leak) and those that cause serious symptoms and require clinical treatments (Clinically relevant postoperative pancreatic fistula (CR-POPF)).

In the present specification, the "prophylaxis" includes prevention of the onset of a disease (whole pathology, or one or plural pathologies), and delay of the onset of the disease. A "prophylactically effective amount" refers to a dose of a lipase inhibitor sufficient to achieve such object.

In the present specification, the "treatment" refers to cure of a disease (whole pathology, or one or plural pathologies), improvement of the disease, and suppression of progression of the severity of the disease. A "therapeutically effective amount" refers to a dose of a lipase inhibitor sufficient to achieve such object.

### (administration route)

In practicing the present invention, the lipase inhibitor can be orally or parenterally administered alone or as a pharmaceutical composition to a subject in need of the administration. As used herein, intraperitoneal administration is preferred, and intraperitoneal local administration is particularly preferable.

### (dose)

In practicing the present invention, the dose of the lipase inhibitor varies depending on the subject of administration, administration route, and age and symptoms of the subject of administration, and is not particularly limited. For example, for intraperitoneal administration to an adult patient (body weight about 40 to 80 kg, for example, 60 kg), the dose can be selected from the range of 1 to 1000 mg per day. It may be administered collectively at the time of surgery, or may be administered multiple times after surgery. The dose of the lipase inhibitor also varies depending on the individual medicaments to be used. Thus, those skilled in the art will readily understand that it can be administered in smaller doses, for example, in the range of 1 to 500 mg per day, or in the range of 1 to 300 mg per day. Therefore, the embodiment of administration of the lipase inhibitor within such range is naturally encompassed in the aforementioned "administration in the range of 1 to 1000 mg".

The prophylactic or therapeutic agent of the present invention can be safely administered to a subject (mammals including humans, especially humans) in need of the administration thereof.

### (administration form)

In practicing the present invention, the lipase inhibitor can be used in any form of a single form or the form of a pharmaceutical composition containing a lipase inhibitor as an active ingredient together with a pharmaceutically acceptable carrier, and the like, as a "prophylactic or therapeutic agent for leakage of pancreatic juice or pancreatic fistula after abdominal surgery" (hereinafter sometimes to be abbreviated as "the prophylactic or therapeutic agent of the present invention"). Preferably, it is used in the form of a pharmaceutical composition.

In practicing the present invention, for example, the dosage form used in this field of the art such as tablet, emulsifier, suspension, controlled-release preparation, hydrogel preparation, or the like can be selected as the pharmaceutical composition. As the "pharmaceutically acceptable carrier", various carriers conventionally used in the field of formulation technology can be used.

The pharmaceutical composition of the present invention can be produced by adding a lipase inhibitor at a proportion of generally 0.001 to 20% (w/w), preferably 0.01 to 10% (w/w), based on the total amount of the preparation, though subject to change depending on the dosage form, administration method, carrier, and the like. The pharmaceutical composition can be produced by a method conventionally used in the field of formulation technology, according to the form thereof.

The embodiments of the pharmaceutical compositions preferred in practicing the present invention are explained below.

### (1) emulsified preparation

The embodiment of one preferred pharmaceutical compositions in practicing the present invention is an "emulsified preparation containing a lipase inhibitor and a dispersing agent".

As the dispersing agent, polyalkylene glycol (e.g., polyethylene glycol, polyoxyethylene polyoxypropylene glycol etc.), ether type nonionic surfactant (e.g., polyoxyethylene alkyl ether, polyoxyethylene alkylphenylether etc.), or the like can be used.

For example, when polyethylene glycol is used, one having a molecular weight in the range of 1000 to 20000 is preferably used.

In practicing the present invention, it is preferable to use an emulsified preparation containing 0.01 to 10% (lipase inhibitor weight/dispersing agent weight) of a lipase inhibitor.

The "emulsified preparation containing a lipase inhibitor and a dispersing agent" of the present invention can be produced by a method widely used in this field of the art and by using a lipase inhibitor, a dispersing agent, and other necessary additives.

### (2) hydrogel preparation

The embodiment of another preferred pharmaceutical composition in practicing the present invention is a "hydrogel preparation containing a complex of a lipase inhibitor and hydrogel".

By forming a complex of a lipase inhibitor and hydrogel (hydrogel preparation), it is possible to prevent leakage of pancreatic juice and to allow the lipase inhibitor to act in a sustained manner over a certain period of time locally in the abdominal cavity (sustained-release preparation).

The hydrogel preparation of the present invention can be appropriately produced using a lipase inhibitor and hydrogel-forming components, according to a technique known in this field of the art and in consideration of a desired duration and the like. Preferred hydrogel-forming components for forming hydrogel include, for example, the following combinations:
1) a combination of a hydrophilic biocompatible polymer and a biocompatible hydrophilic polymer crosslinking agent (hydrogel-forming component 1)
2) a combination of a synthetic biocompatible polymer having a functional group (polymer A) and a synthetic biocompatible polymer having a functional group that reacts with polymer A under in vivo conditions (polymer B) (hydrogel-forming component 2), or
3) a combination of a low-molecular-weight compound having a functional group and a biocompatible hydrophilic polymer crosslinking agent (hydrogel-forming component 3).

### hydrogel-forming component 1

As the "hydrophilic biocompatible polymer", proteins, polysaccharides, and non-biological polymers, and derivatives thereof and combinations thereof can be mentioned.

The proteins can be selected from the group consisting of gelatin, collagen, albumin, hemoglobin, fibrinogen, fibrin, casein, fibronectin, elastin, keratin, and laminin, and derivatives thereof and combinations thereof.

The polysaccharides can be selected from the group consisting of glycosaminoglycan, starch, cellulose, dextran, hemicellulose, xylan, agarose, alginate, and chitosan, and derivatives thereof and combinations thereof.

The non-biological polymers can be selected from the group consisting of polyacrylate, polymethacrylate, polyacrylamide, polymethacrylamide, polyethyleneimine, polyvinyl resin, polylactide-glycolide, polycaprolactone, and polyoxyethylene, and derivatives thereof and combinations thereof.

The "hydrophilic biocompatible polymer" may be crosslinked. The crosslinking can be performed by a method known in this field of the art.

While the hydrophilic biocompatible polymer is not limited, at least one selected from gelatin, collagen, fibrinogen, fibrin, cellulose, and chitosan, and derivatives thereof is preferred, at least one selected from crosslinked gelatin, fibrinogen, and fibrin, and derivatives thereof is more preferred, and at least one selected from fibrinogen, and fibrin, and derivatives thereof is particularly preferred. In addition, crosslinked gelatin is also particularly preferred.

As the "biocompatible hydrophilic polymer crosslinking agent", a hydrophilic polymer crosslinking agent can be mentioned. Preferable examples thereof include a polyalkylene oxide polymer having a functional group selected from the group consisting of a succinimidyl ester (-CON(COCH₂)₂), an NHS-ester group, an imide ester group, an aldehyde group, a carboxy group in the presence of a carbodiimide, an isocyanate, a THPP (beta-[tris(hydroxymethyl)phosphino]propionic acid), a maleimide, a haloacetyl, a thiol group, an amino group, an azido group, an alkyne group including an ethynyl group and the like, a cyclic alkyne group including a cyclooctyne group and the like, and mixtures thereof.

As the hydrophilic polymer crosslinking agent having such functional group, a polyethylene glycol having a functional group selected from a succinimidyl ester (-CON(COCH₂)₂), an aldehyde(-CHO), a thiol group, an amino group, an azido group, an alkyne group including an ethynyl group and the like, a cyclic alkyne group including a cyclooctyne group and the like, and an isocyanate (-N=C=O) is particularly preferred.

In this embodiment, the "hydrophilic biocompatible polymers" are crosslinked by a "biocompatible hydrophilic polymer crosslinking agent", and bonded to each other. They constitute a network to form a gel which is provided as a hydrogel. This gelling reaction may also be performed by appropriately adding other components widely used in this field of the art.

Those of ordinary skill in the art can perform the above production step according to a conventional method for producing hydrogel in this field of the art.

### hydrogel-forming component 2

The "synthetic biocompatible polymer having a functional group (polymer A)" includes a polyalkylene oxide having two or more reactive groups, and examples of the functional group include those recited as examples in the above-mentioned hydrophilic polymer crosslinking agent.

As the polyalkylene oxide, for example, a polyethylene oxide or a derivative thereof is preferred.

The "synthetic biocompatible polymer having a functional group that reacts with polymer A under in vivo conditions (polymer B)" includes a polyalkylene oxide having two or more reactive groups, and examples of the functional group include those recited as examples in the above-mentioned hydrophilic polymer crosslinking agent.

As the polyalkylene oxide, for example, a polyethylene oxide or a derivative thereof is preferred. When a polyethylene oxide or a derivative thereof is used, one having a molecular weight in the range of 1000 to 80000 is preferably used.

In this embodiment, the "synthetic biocompatible polymer having a functional group (polymer A)" and the "synthetic biocompatible polymer having a functional group that reacts with polymer A under in vivo conditions (polymer B)" are bonded to each other between the functional groups thereof. They constitute a network to form a gel which is provided as a hydrogel. This gelling reaction may also be performed by appropriately adding other components widely used in this field of the art.

Those of ordinary skill in the art can perform the above production step according to a conventional method for producing hydrogel in this field of the art.

### hydrogel-forming component 3

The "low-molecular-weight compound having a functional group" is a compound having one or more kinds of two or more functional groups, and the functional group includes those recited as examples of the functional group in the above-mentioned hydrophilic polymer crosslinking agent.

For example, an amino acid or oligopeptide having an amino group, a thiol group, and the like, a polyamine such as spermine and the like, a diamine, a triamine compound such as ethylenediamine, diethylenetriamine, and the like, and the like can be mentioned.

As the "biocompatible hydrophilic polymer crosslinking agent", those recited as examples of the "biocompatible hydrophilic polymer crosslinking agent" in the above-mentioned hydrogel-forming component 1 can be mentioned.

In this embodiment, the "low-molecular-weight compound having a functional group" and the "biocompatible hydrophilic polymer crosslinking agent" are crosslinked by a "biocompatible hydrophilic polymer crosslinking agent", and bonded to each other. They constitute a network to form a gel which is provided as a hydrogel. This gelling reaction may also be performed by appropriately adding other components widely used in this field of the art.

Those of ordinary skill in the art can perform the above production step according to a conventional method for producing hydrogel in this field of the art.

The hydrogel preparation of the present invention can be produced according to a conventional method for producing hydrogel including dissolving (emulsifying/suspending), mixing, stirring, and the like, of a lipase inhibitor, the above-mentioned hydrogel-forming components, and other components widely used in this field of the art, and can be used for two-component extrusion or spraying, or as a hydrogel sheet. For example, each component forming the above-mentioned hydrogel is premixed as an aqueous solution, and then extruded and sprayed to form a hydrogel. Alternatively, a hydrogel can also be formed by extruding with a mixing tip from a syringe capable of individually encapsulating two-component aqueous solutions and spraying. Furthermore, a hydrogel sheet is formed in advance from the hydrogel-forming components and directly attached to the affected area, or a hydrogel sheet is impregnated with a lipase inhibitor and then used. Furthermore, a hydrogel sheet is formed in advance from the hydrogel-forming components, powderized by freeze-drying or the like or dried into a dry sheet, and then the powder is swollen with an aqueous solution containing a lipase inhibitor to form a hydrogel sheet, which is used by directly attaching to the affected area.

In addition, the hydrogel preparation can also be produced after previously formulating the lipase inhibitor as an "emulsified preparation containing a lipase inhibitor and a dispersing agent" described in the above-mentioned section of (emulsified preparation).

When the lipase inhibitor is poorly soluble in water, hydrogel can also be produced after leading the lipase inhibitor into a water-soluble form by other known method in the art (e.g., encapsulation in polymer micelle) and using same as a "lipase inhibitor".

In practicing the present invention, it is preferable to use a hydrogel preparation containing 0.01 to 5% (lipase inhibitor weight/hydrogel-forming components weight) of a lipase inhibitor.

### (combined use with other agents)

The prophylactic or therapeutic agent of the present invention can be combined with other medicaments and used for the prophylaxis or treatment of leakage of pancreatic juice or pancreatic fistula after abdominal surgery, and the combined use with other medicaments is expected to achieve a superior prophylactic or therapeutic effect. Such combination therapy is also expected to lower the dose of other medicaments and reduce the side effects they have.

### [Example]

The present invention is explained in more detail in the following by referring to Examples, which are not to be construed as limiting the scope of the present invention in any manner. Unless particularly indicated, the reagents, apparatuses, and materials to be used in the present invention are commercially available, or can be appropriately prepared by those of ordinary skill in the art.

### [Experiment materials and experiment methods]

### <Experiment materials>

Cetilistat was purchased from Tokyo Chemical Industry Co., Ltd. Polyethylene glycol (molecular weight 6000) (hereinafter sometimes to be abbreviated as PEG in Examples) was provided by NOF CORPORATION (Tokyo, Japan).

### <Preparation of PEG-cetilistat-containing emulsion>

A PEG-cetilistat-containing emulsion was prepared by the following procedure.
1) Cetilistat (50 mg) and PEG (1500 mg) (both ambient temperature, solid and powdery) were blended by stirring at room temperature for 5 min.
2) Both components were blended by stirring in a warm bath (75°C) for 15 min.
3) To the mixture was added DDW (deionized distilled water) (3450 mg), and the mixture was further blended by stirring in a warm bath (75°C) for 15 min.
4) 1 ml of the obtained emulsion was separated (1 ml=1140 mg, RT) and diluted 11.4-fold (10.4 ml of DDW was added) to give a cloudy emulsion of cetilistat (concentration 1 mg/ml)) (hereinafter sometimes to be abbreviated as PEG-C in the Examples).

### <Study of Pancreatic acinar cell-protecting effect (in vitro) of PEG-C>

### (Collection of cell)

Primary acinar cells were collected by the following procedure.
1) SD rats (9- to 12-week-old, Shimizu Laboratory Supplies Co., Ltd.) were euthanized and the blood was removed.
2) A medium containing 2 mg/ml of collagenase D (Roche, derived from Clostridium histolyticum, 0.24 U/mg) was injected into the rat bile duct.
3) The pancreas was isolated and incubated in 15 ml of collagenase D-containing medium at 37°C for about 20 min.
4) Then, the solution was shaken for 20 sec to give a cell suspension.
5) The suspension was filtered through a 1 mm metal mesh and centrifuged at 100xg for 3 min, and the supernatant was discarded.
6) The precipitate was suspended again in the medium (50 ml), filtered with a cell strainer 352350 having 70 µm mesh (Falcon), and centrifuged at 100×g for 3 min. The supernatant was discarded and the desired cells were recovered.

### (Cell seeding)

It was dispensed by 1 ml to each well of a 48-well plate (polystyrene, no surface treatment, Asahi Glass Co., Ltd. (AGC)) at a concentration of about 1.5×10⁵ cells/ml. It is difficult to accurately determine the cell density because the cells are separated into the state of mulberry fruit at the time of cell collection. However, the experiment was conducted by dispensing the cell suspension such that each well certainly had the same density.

The composition of the medium (per 1000 ml) used in the above-mentioned experiment was as follows.
Krebs-Ringer bicarbonate buffer (K4002-1 L, Sigma-Aldrich): one calcium chloride: 1 mM
HEPES: 10 mM
sodium hydrogen carbonate: 10 mM
bovine serum albumin (fatty acid-free) (08587-84, Nacalai Tesque): 1000 mg
trypsin inhibitor (derived from soybean) (202-09221, FUJIFILM Wako Pure Chemical Corporation): 100 mg

### (Blending neutral fats and PEG-C with medium)

A stock solution was prepared by dissolving triolein (208-02981, FUJIFILM Wako Pure Chemical Corporation) in DMSO (13408-64, Nacalai Tesque) (triolein concentration: 50 mM), 0.5 ml of a medium in which the triolein-DMSO stock solution and PEG-C were dissolved to two times the final concentration and 0.5 ml of the cell suspension were combined into 1 ml, which was dispensed to the well to achieve the desired concentration.

### (LDH assay)

100 µl of the supernatant was collected at each time point, the absorbance was measured using the Cytotoxicity LDH Assay Kit-WST (Dojindo Laboratories Co., Ltd.) according to the manual, and the lactic acid dehydrogenase (LDH) activity was measured.

### <Animal experiments>

### (Animal)

All animal experiments were approved by the Institutional Animal Care and Use Committee of Kyoto University and conducted in accordance with the ARRIVE guidelines.

Obese rats were created according to previous reports. The outline is as follows.

3-Week-old male Sprague-Dawley rats (Shimizu Laboratory Supplies Co., Ltd.) were allowed to freely take a high-fat diet (D12451, Research Diets Inc) for not less than 6 weeks. Rats weighing 400-450 g were used. Male animals were used to minimize variation in the body weight and biochemistry. The rats were bred at a temperature of 21-25°C in a 12-hour light-dark cycle and were allowed to drink freely.

### (Creation of pancreatic fistula model rat and treatment of adipose tissue damage; see Fig. 1)

A pancreatic fistula model rat (PT group) was created by pancreatic dissection according to previous reports. The outline is as follows.

Rats were anesthetized by inhalation of sevoflurane and intraperitoneal injection of pentobarbital. The pain was relieved by subcutaneous injection of buprenorphine. After laparotomy of the median line, the pancreas was secured ventral to the portal vein without passive pancreas. The splenic artery and vein were preserved. The rat pancreatic duct is composed of four parts: gastric duct, duodenal duct, common duct, and splenic duct. The splenic duct and the surrounding pancreatic parenchyma were cut with a sword at the level just above the portal vein. Bleeding from the cut end of the pancreas was stopped by natural compression of gauze. Attention was paid not to damage the adipose tissue.

To clarify the effect of the damage of the adipose tissue, a part of the animals underwent a treatment of the adipose tissue damage (the treatment symbolized as +F). The adipose tissue around the epididymis on both sides was dissected, placed in a 35 mm polystyrene sterile dish, and immersed in 1 ml of saline. Adipose tissue was coagulated for 5 min using a biopolar electrocautery system (ConMedSaverGenesis) (output 50 W). After cooling at room temperature for 3 min (to avoid burns), the damaged adipose tissue and exudate were returned to the abdominal cavity (PT+F group).

### (Animal grouping and treatment)

The experiment animals were divided into groups, and the following treatments were performed on each group.
Sham group: Only single laparotomy was performed.
Sham+F group: Single laparotomy and fat damage treatment were performed.
PT group: Only pancreatic dissection operation was performed. PT+F group: Pancreatic dissection operation and fat damage treatment were performed.
PT+F+C: After the pancreatic dissection operation, PEG-C was administered (PEG-C; 1 ml intraperitoneal administration), and a fat damage treatment was performed.
PT+F+V: After the pancreatic dissection operation, PEG aqueous solution was administered, and a fat damage treatment was performed (PEG aqueous solution; 1 ml intraperitoneal administration; PEG content was equal to that in the case of PEG-C.)

### (Blood sample collection)

The rats were sacrificed 24 hr after the surgery or observed for 7 days for survival analysis.

Blood samples were collected from the inferior vena cava immediately after death and the serum was separated. The abdominal cavity washing was irrigated with 4 ml of saline, then collected from the abdominal cavity, and centrifuged to obtain a supernatant. The supernatant was subjected to evaluation as ascites. All ascites and serum samples were immediately analyzed or cryopreserved at -80°C for later analysis and used only once after thawing.

### (Biochemical assay)

The activities of amylase and lipase were determined at a specialized clinical laboratory (Japan Clinical Laboratories, Inc.)

### (Measurement of free fatty acid concentration)

The free fatty acid concentration of each blood sample was determined by high performance liquid chromatography using a labeling agent (XSRFAR01; YMC. CO., LTD.) (detector: Prominence SPD-20A, SHIMADZU Corporation; column: YMC-Pack FA, YMC. CO., LTD.). Margaric acid (C17:0) was used as an internal standard. The total free fatty acid (TFA) concentration was calculated by adding individual C12:0, C14:0, C16:0, C18:0, C16:1, C18:1, and C18:2 fatty acids. The unsaturated fatty acid concentration was calculated according to previous reports by adding individual C16:1, C18:1, and C18:2 fatty acids.

### (Histological analysis)

After sacrifice, the tissue was excised and fixed with 10% buffered formalin for 48 hr to prepare a 4 µm-thick paraffin-embedded section. Hematoxylin and eosin staining, and alizarin red S staining (Sigma-Aldrich) for detection of calcium deposition were performed according to standard protocols.

### (Statistical analysis)

After applying the Levene's variance test, each group was compared using the t-test, Mann-Whitney U test, and one-way ANOVA with multiple comparison Tukey's test, or Steel-Dwass test as appropriate. The categorical variables were compared using Fisher's exact probability test. The numerical values of the experiment results show average values. Five independent animals/samples were analyzed unless otherwise indicated. Survival analysis was performed using the Kaplan-Meier plot for evaluation by the method of Logrank test. The data analysis was performed using JMP Pro version 14.0 (SAS).

### [Example 1: Evaluation of effect of fat damage on pancreatic fistula model mouse]

As described above, the production of pancreatic fistula model mice (pancreatic dissection treatment) and fat damage treatment were performed according to the schematic diagram shown in Fig. 1.
(1) In the PT group, which is a mild pancreatic fistula-like model, no notable changes were observed in the abdominal cavity as compared with the Sham group, and the fat damage treatment (+F) to the Sham group did not produce notable changes (Sham+F group). On the other hand, in the PT+F group in which the both treatments were combined, a remarkable white deposit was formed in the abdominal cavity (Fig. 2).
(2) Free fatty acid concentration (µM) in ascites of each group was examined.
   In the PT+F group, it was found that lipolysis occurred highly in the abdominal cavity and free fatty acid was produced. No significant increase in free fatty acid production was observed compared with the control group by pancreatic dissection treatment alone or fat damage treatment alone (Fig. 3) .
(3) Pancreatic enzyme activity (IU/L) in ascites was examined.
   In the PT group, significant leakage of the pancreatic juice occurred in the abdominal cavity. In the PT+F group, a significantly higher degree of pancreatic juice leakage was observed as compared with the PT group, and a significant enhancement of pancreatic enzyme activity in ascites was observed (Fig. 4).
(4) histological findings of pancreas were examined.
   In the PT group, mild edema and infiltration of inflammatory cells were observed in the pancreas. Similar changes were observed in the PT+F group. In the PT+F group, attachment of glass-like deposits to the surface of the pancreas and infiltration of inflammatory cells around the deposits were observed. This deposit was stained orange by alizarin S staining and was considered to be a deposit of fatty acid calcium (Fig. 5).
(5) Survival rates of PT group and PT+F group were examined.
   No death cases were observed in the PT group after 1 week of observation. A significant decrease in the survival rate was observed in the PT+F group, indicating more severe cases (Fig. 6).

### [Example 2: Evaluation of effect of lipase inhibitor (in vitro)]

(1) Since pancreatic acinar cells produce and secrete pancreatic enzymes including lipase, neutral fats are decomposed into free fatty acids in such environment. In order to verify the effect of neutral fats on pancreatic acinar cells, neutral fat (triolein) was administered in the medium and cytotoxicity was verified.
   A time-course increase in the LDH concentration in the supernatant was observed depending on the concentration of neutral fat in the medium, and enhancement of cytotoxicity over time was confirmed (Fig. 7). This change is characteristic of pancreatic acinar cells and was not observed at all in similar experiments using other type of cells (Rat MSC (Mesenchymal stem cell)).
(2) It was confirmed that administration of PEG-C in the medium reduces damage on pancreatic acinar cells even in the presence of neutral fats.
   As shown in Fig. 8, a pancreatic acinar cell-protecting effect was observed in the PEG-C use group in a dose-dependent manner as compared with the control group (without PEG-C addition). In particular, in the PEG-C 100 µM administration group, a significant decrease in the concentration of oleic acid in the medium was observed.

### [Example 3: Evaluation of effect of lipase inhibitor (in vivo)]

(1) Therapeutic effect of PEG-C on pancreatic fistula model mouse was evaluated (intraperitoneal findings).
   When intraperitoneal changes were confirmed, the deposition of the intraperitoneal white deposits observed in the PT+F group was suppressed by the administration of PEG-C (PT+F+C group). By vehicle administration (PEG aqueous solution) alone, the same changes as in the PT+F group were observed (PT+F+V group) (Fig. 9).
(2) Pancreatic enzyme activity in ascites (IU/L) was examined.
   A significant decrease in the leakage of pancreatic juice was confirmed in the PT+F+C group compared with the PT+F group. On the other hand, this effect was not observed in the vehicle administration group (PT+F+V group) (Fig. 10).
(3) The concentration (µM) of free fatty acid in ascites was examined. It was clarified that the concentration of free fatty acid in ascites is significantly low in the PT+F+C group compared with the PT+F group, and production of free fatty acid is significantly suppressed (Fig. 11).
(4) Survival rate of each group was verified.
   No death case was observed in the PT+F+C group during the observation period, and it was confirmed that the survival rate is significantly improved compared with the PT+F group (Fig. 12) .

### (Brief Summary)

From the results of verification from various aspects as shown in Examples 1 to 3, it was clarified that intraperitoneal administration of PEG-C can suppress the aggravation of pancreatic fistula through suppression of intraperitoneal lipolysis.

Lipase inhibitors contribute to effective prophylaxis and treatment strategies for pancreatic fistula.

### [Example 4: Preparation of cetilistat-hydrogel complex]

### <Hydrogel-forming components>

As hydrogel-forming components, SUNBRIGHT PTE-100GS (molecular weight 10,000, end is succinimidyl glutarate) (NOF CORPORATION) and SUNBRIGHT PTE-100SH (molecular weight 10,000, end is thiol) (NOF CORPORATION) which are commercially available tetrafunctional group-type polyethylene glycol modifiers were used.

### <Preparation of PEG gel-adjusted solution>

A solution (pH 6.0) in which SUNBRIGHT PTE-100GS was dissolved in dilute hydrochloric acid to a concentration of 20% (w/v) was prepared (solution 1). On the other hand, a solution (pH 9.6) in which SUNBRIGHT PTE-100SH was dissolved in phosphoric acid-carbonic acid buffer to a concentration of 20%(w/v) was prepared (solution 2). Solution 1 and solution 2 were placed in a 4 ml DIY dual syringe manufactured by Nordson, and a mixable needle outlet was attached to give a PEG gel-adjusted solution.

Hydrogel is rapidly formed by extruding each solution with the dual syringe, (hereinafter sometimes to be abbreviated as PEG gel in this Example).

### <Preparation of cetilistat-hydrogel complex-adjusted solution>

Cetilistat and SUNBRIGHT PTE-100GS were mixed in a powder state at a ratio of 1:50 (weight ratio), stirred for 5 min in a warm bath at 75°C, and then dilute hydrochloric acid was added to SUNBRIGHT PTE-100GS to a concentration of 20% (w/v) to prepare a cloudy emulsion-like solution 3 (pH 6.0).

On the other hand, SUNBRIGHT PTE-100SH was added to a phosphate-carbonic acid buffer solution to a concentration of 20% (w/v) to prepare a solution 4 (pH 9.6).

Solution 3 and solution 4 were placed in a 4 ml DIY dual syringe manufactured by Nordson, and a mixable needle outlet was attached to prepare a cetilistat-hydrogel complex-adjusted solution.

A hydrogel containing cetilistat is rapidly formed by extruding each solution with the dual syringe (hereinafter sometimes to be abbreviated as PEG gel-C in this Example).

In the hydrogel, the cetilistat concentration is 2.5 mg/ml (corresponding to specific gravity of about 1; 2.5 mg/g), and the dose of cetilistat when 500 µl of the hydrogel is administered around the pancreas of an animal is 1.25 mg.

### [Example 5: Evaluation of effect of PEG gel-C (in vivo)]

In order to evaluate the effect of PEG gel-C on pancreatic fistula, an animal experiment was performed using a pancreatic fistula model mouse.

### <Experiment method>

As the experimental animal, the same animal as described in the above-mentioned section of <Animal experiment> (animal) was used.

The animal experiment was conducted according to the following procedure.
1. On the day of the experiment, a PEG gel-adjusted solution and a cetilistat-hydrogel complex-adjusted solution were prepared.
2. The rat pancreas was dissected under systemic anesthesia, and after hemostasis (similar to the <animal experiment> PT group), 500 µl of the PEG gel-adjusted solution or cetilistat-hydrogel complex-adjusted solution was administered around the pancreas (PEG gel (N=2), PEG gel-C (N=3), PEG gel-C group: dose of cetilistat was 1.25 mg/body).
3. Completion of gelation after administration of the drug solution (about 1 min after administration) was confirmed.
4. The fat damage treatment shown in Fig. 1 was performed, and the fat was cauterized and coagulated, and the exudate was returned to the abdominal cavity (+F treatment), and then the surgical wound was closed.
5. Rats were sacrificed 24 hr after surgery, tissues were removed and subjected to histological analysis.

Grouping of animals in this animal experiment was as follows.
PEG gel-C group: group administered with PEG gel-C (N=3) in the above-mentioned animal experiment.
PEG gel group: group administered with PEG gel (N=2) in the above-mentioned animal experiment.
PT group: group in which only pancreatic dissection operation was performed (see data obtained in the animal experiment performed in Example 1) (N=5).
PT+F group: non-treatment group without administration of both PEG gel-C and PEG gel (see data obtained in the animal experiment performed in Example 1) (N =5) .

### <Experiment results>

### (1) Study of histological findings

### (PEG gel-C administration group)

As shown in Fig. 13 and Fig. 14, a clear decrease (suppression) was confirmed in the inflammatory findings and saponification in the whole abdominal cavity in all of the PEG gel-C groups, compared with the non-treatment group (see Fig. 17) .

### (PEG gel administration group)

As shown in Fig. 15 and Fig. 16, in the PEG gel group, no clear change was confirmed in the findings in the whole abdominal cavity, compared with the non-treatment group (see Fig. 17) .

### (2) Study of pancreatic enzyme activity (IU/L) in ascites

The measurement results are shown in Fig. 18. In the PEG gel-C group, it was confirmed that the leakage of pancreatic enzyme into the ascites was significantly suppressed compared with the non-treatment group (PT+F group). For the PT group and the PT+F group, the data obtained in the aforementioned Example 1 was used.

### (Brief Summary)

It was confirmed that the aggravation of pancreatic fistula may be suppressed by covering the pancreas with a cetilistat-hydrogel complex (PEG gel-C).

A hydrogel preparation containing a lipase inhibitor provides a new therapeutic strategy for pancreatic fistula by efficiently distributing the lipase inhibitor locally in the pancreas.

### [Example 6: Preparation of orlistat-hydrogel complex]

The same operation as in the preparation method described in Example 4 <Preparation of cetilistat-hydrogel complex-adjusted solution> was performed except that orlistat and SUNBRIGHT PTE-100GS were blended in a powder state at a ratio of 1:30 (weight ratio) instead of blending cetilistat and SUNBRIGHT PTE-100GS in a powder state at a ratio of 1:50 (weight ratio) to obtain an orlistat-hydrogel complex-adjusted solution containing orlistat.

A hydrogel containing orlistat is rapidly formed by extruding each solution with the dual syringe (hereinafter sometimes to be abbreviated as PEG gel-O in this Example).

In the hydrogel, the orlistat concentration is 3.3 mg/ml (corresponding to specific gravity of about 1; 3.3 mg/g), and the dose of orlistat when 500 µl of the hydrogel is administered around the pancreas of an animal is 1.67 mg.

### [Example 7: Evaluation of effect of PEG gel-O (in vivo)]

The same operation as in Example 5 was performed except that PEG gel-O was used instead of PEG gel-C, and the effect on pancreatic fistula was evaluated.

Grouping of animals in this animal experiment was as follows.

### PEG gel-O group: group administered with PEG gel-O (N=2) in the above-mentioned animal experiment

### <Experiment results>

### (1) Study of histological findings

### (PEG gel-O administration group)

As shown in Fig. 19, a clear decrease (suppression) was confirmed in the inflammatory findings and saponification in the whole abdominal cavity in the PEG gel-O group, compared with the non-treatment group (see Fig. 17).

### (2) Study of pancreatic enzyme activity (IU/L) in ascites

Ascitic amylase was measured as the pancreatic enzyme activity. As a result, the PEG gel-O group showed 3559IU/L, and it was confirmed that the leakage of pancreatic enzyme into the ascites was significantly suppressed compared with the non-treatment group (PT+F group). For the PT group and the PT+F group, the data obtained in the aforementioned Example 1 (16868IU/L) was used.

### (Brief Summary)

It was confirmed that the aggravation of pancreatic fistula may be suppressed by covering the pancreas with an orlistat-hydrogel complex (PEG gel-O).

### [Example 8: Preparation of cetilistat-bolheal (fibrin gel) complex]

### <Hydrogel-forming components>

As the hydrogel-forming component, TEIJIN PHARMA bolheal tissue adhesion 1 ml preparation^{note 1} (hereinafter sometimes to be abbreviated as bolheal) was used. note 1) bolheal tissue adhesion preparation

The bolheal tissue adhesion preparation is composed of a fibrinogen freeze-dry powder, a fibrinogen dissolving solution, a thrombin freeze-dry powder, and a thrombin dissolving solution, and the detail is as follows.
vial 1: fibrinogen freeze-dry powder (containing fibrinogen and blood coagulation factor XIII derived from human plasma)
vial 2: fibrinogen dissolving solution (containing aprotinin derived from bovine lung)
vial 3: thrombin freeze-dry powder (containing thrombin derived from human plasma)
vial 4: thrombin dissolving solution (containing calcium chloride hydrate)

By using the bolheal tissue adhesion preparation as a hydrogel-forming component, trombin acts on fibrinogen, which is a glycoprotein in plasma, to produce fibrin monomer, and the fibrin monomer is further polymerized by the action of calcium to form fibrin polymer. Furthermore, the fibrin polymers are crosslinked by the action of factor XIII to form a mesh-like fiber called stabilized fibrin, whereby a so-called fibrin gel is produced.

### <Preparation of cetilistat-bolheal complex-adjusted solution>

0.12 mL of PEG-C adjusted in the aforementioned Example and 0.88 mL of thrombin solution for bolheal were mixed to prepare a thrombin solution containing PEG-C. The thrombin solution was added to the thrombin freeze-dry powder, whereby a thrombin solution containing PEG-C was prepared.

On the other hand, a fibrinogen dissolving solution was added to the fibrinogen freeze-dry powder according to the method described in the package insert of "bolheal tissue adhesion 1 ml preparation" to prepare a fibrinogen solution.

The above-mentioned thrombin solution containing PEG-C and the fibrinogen solution were added to a dual syringe attached to bolheal, and it was confirmed that a hydrogel containing cetilistat is rapidly formed by extruding each solution with the dual syringe (hereinafter sometimes to be abbreviated as bolheal-C in this Example).

In the hydrogel, the cetilistat concentration is 2.0 mg/ml (corresponding to specific gravity of about 1; 2.0 mg/g), and the dose of cetilistat when 500 µl of the hydrogel is administered around the pancreas of an animal is 1.0 mg.

### [Example 9: Evaluation of effect of bolheal-C (in vivo)]

The same operation as in Example 5 was performed except that bolheal-C was used instead of PEG gel-C, and the effect on pancreatic fistula was evaluated.

Grouping of animals in this animal experiment was as follows.
bolheal-C group: group administered with bolheal-C (N=2) in the above-mentioned animal experiment

### <Experiment results>

### (1) Study of histological findings

### (Bolheal-C administration group)

As shown in Fig. 20, a decrease (suppression) was confirmed in the inflammatory findings and saponification in the whole abdominal cavity in the bolheal-C group, compared with the non-treatment group (see Fig. 17).

### (Brief Summary)

It was confirmed that the aggravation of pancreatic fistula may be suppressed by covering the pancreas with a cetilistat-bolheal complex (bolheal-C).

### [Comparative Example 1: Preparation of bolheal alone]

In the preparation method described in Example 8 <Preparation of cetilistat-bolheal complex-adjusted solution>, the same operation was performed except that thrombin dissolving solution for bolheal was used instead of the thrombin dissolving solution containing PEG-C, and a hydrogel consisting of bolheal alone and not containing cetilistat was prepared (hereinafter sometimes to be abbreviated as bolheal alone in this Example).

### [Comparative Example 2: Evaluation of effect of bolheal alone (in vivo)]

The same operation as in Example 5 was performed except that bolheal alone was used instead of PEG gel-C, and the effect on pancreatic fistula was evaluated.

Grouping of animals in this animal experiment was as follows.
bolheal alone group: group administered with bolheal alone (N=2) in the above-mentioned animal experiment.

### <Experiment results>

### (1) Study of histological findings

### (Bolheal alone administration group)

As shown in Fig. 21, inflammatory findings and saponification in the whole abdominal cavity similar to those in the non-treatment group (see Fig. 17) were confirmed in the bolheal alone group.

### (Summary)

It was clarified that lipase inhibitors contribute to effective prophylaxis and treatment strategies for pancreatic fistula. It was also clarified that a hydrogel preparation of a lipase inhibitor is one of the effective administration methods thereof.

### [Industrial Applicability]

The present invention provides a prophylactic or therapeutic agent for the leakage of pancreatic juice and/or pancreatic fistula after abdominal surgery, and a method for preventing or treating the leakage of pancreatic juice or pancreatic fistula after abdominal surgery. The present invention is useful in the pharmaceutical field.

This application is based on patent application Nos. 2020-18309 filed in Japan (filing date: February 5, 2020) and 2020-167881 filed in Japan (filing date: October 2, 2020), the contents of which are incorporated in full herein.

## Claims

1. A prophylactic or therapeutic agent for the leakage of pancreatic juice and/or pancreatic fistula after abdominal surgery, comprising a lipase inhibitor as an active ingredient.

2. The prophylactic or therapeutic agent according to claim 1 for the prophylaxis or treatment of pancreatic fistula after abdominal surgery.

3. The prophylactic or therapeutic agent according to claim 2, wherein the pancreatic fistula is clinically relevant postoperative pancreatic fistula (CR-POPF).

4. The prophylactic or therapeutic agent according to any one of claims 1 to 3, wherein the abdominal surgery is a pancreatic surgery.

5. The prophylactic or therapeutic agent according to any one of claims 1 to 4, wherein the lipase inhibitor is one medicament or a combination of two or more medicaments, selected from orlistat, cetilistat, lalistat, and atglistatin.

6. The prophylactic or therapeutic agent according to claim 5, wherein the lipase inhibitor is one medicament or a combination of two medicaments, selected from orlistat and cetilistat.

7. The prophylactic or therapeutic agent according to any one of claims 1 to 6, wherein 0.01 to 20%(w/w) of the lipase inhibitor is contained.

8. The prophylactic or therapeutic agent according to any one of claims 1 to 7, wherein the agent is an emulsified preparation comprising a lipase inhibitor and a dispersing agent.

9. The prophylactic or therapeutic agent according to any one of claims 1 to 7, wherein the agent is a hydrogel preparation comprising a complex of a lipase inhibitor and a hydrogel.

10. The prophylactic or therapeutic agent according to claim 9, wherein the hydrogel is a hydrogel preparation formed from any one of the following combinations of hydrogel-forming components:
1) a combination of a hydrophilic biocompatible polymer and a biocompatible hydrophilic polymer crosslinking agent;
2) a combination of a synthetic biocompatible polymer having a functional group (polymer A) and a synthetic biocompatible polymer having a functional group that reacts with polymer A under in vivo conditions (polymer B); or
3) a combination of a low-molecular-weight compound having a functional group and a biocompatible hydrophilic polymer crosslinking agent.

11. The prophylactic or therapeutic agent according to claim 10, wherein the synthetic biocompatible polymer comprises a polyalkylene oxide having a functional group.

12. The prophylactic or therapeutic agent according to claim 10, wherein the hydrophilic biocompatible polymer is at least one selected from gelatin, collagen, fibrinogen, fibrin, cellulose, and chitosan, and derivatives thereof.

13. The prophylactic or therapeutic agent according to claim 10, wherein the hydrophilic biocompatible polymer is at least one selected from crosslinked gelatin, fibrinogen, and fibrin, and derivatives thereof.

14. The prophylactic or therapeutic agent according to any one of claims 9 to 13, wherein 0.01 to 10%(w/w) of the lipase inhibitor is contained.

15. The prophylactic or therapeutic agent according to claim 14, wherein 0.01 to 5% (lipase inhibitor weight/hydrogel-forming component weight) of the lipase inhibitor is contained.

16. The prophylactic or therapeutic agent according to any one of claims 9 to 15, further comprising a dispersing agent.
